(19) Europäisches Patentamt
European Patent Office
Office européen des brevets

(11) **EP 2 601 923 B1**

(12) **EUROPÄISCHE PATENTSCHRIFT**

(45) Veröffentlichungstag und Bekanntmachung des Hinweises auf die Patenterteilung:
**14.09.2016 Patentblatt 2016/37**

(51) Int Cl.:
*A61F 13/56* (2006.01)     *A61F 13/58* (2006.01)
*A61F 13/62* (2006.01)

(21) Anmeldenummer: **12006673.3**

(22) Anmeldetag: **24.09.2012**

(54) **Windelverschlussband, Bandmaterial, Kreuzwicklung sowie Verfahren zum Wiederverschließen einer Windel**

Nappy closing strip, strip material, cross-wrapping and method for reclosing a nappy

Bande de fermeture de couche, matériau de bande, bobinage croisé et procédé destiné à refermer une couche

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.12.2011 DE 102011120077**

(43) Veröffentlichungstag der Anmeldung:
**12.06.2013 Patentblatt 2013/24**

(73) Patentinhaber: **Lohmann-koester GmbH & Co. KG 96146 Altendorf (DE)**

(72) Erfinder: **Drozdziok-Weinhard, Magdalena 91056 Erlangen (DE)**

(74) Vertreter: **Reuther, Martin**
**Patentanwalt**
**Zehnthofstrasse 9**
**52349 Düren (DE)**

(56) Entgegenhaltungen:
**EP-A1- 0 894 448      EP-A1- 1 000 598**
**EP-A1- 1 629 813      EP-A2- 0 832 631**
**EP-A2- 0 941 728      WO-A1-98/27922**
**DE-A1- 19 805 576     US-A- 5 019 073**
**US-A- 5 176 671       US-A1- 2003 130 644**

EP 2 601 923 B1

## Beschreibung

Die Erfindung betrifft ein Windelverschlussband, ein entsprechendes Bandmaterial sowie ein Verfahren zum Verschließen und Wiederverschließen einer Windel.

Ein gattungsgemäßes Windelverschlussband offenbart die DE 198 05 576 A1, wobei bei diesem Windelverschlussband der männliche Teil eines mechanischen Schließsystems mit einer Klebstoffbeschichtung versehen und mit einem weiblichen Teil des mechanischen Schließsystems mechanisch verbunden ist und wobei bei diesem Windelverschlussband der weibliche Teil an einem Trägerband des Windelverschlussbands fest appliziert ist. Bei der Fertigung der Windel wird das Windelverschlussband bzw. das Trägerband um ein Windelohr einer Windel gelegt, so dass der männliche Teil des mechanischen Schließsystems an der Innenseite der Windel über die Klebstoffbeschichtung fest appliziert ist. Beim Zurückklappen des Windelverschlussbandes, was zum Verschließen der Windel dann notwendig ist, verbleibt die Klebstoffbeschichtung mit dem männlichen Teil an der Innenseite des Windelohres und ermöglicht eine Befestigung der Innenseite des hinteren Windelohres an einem auf der Vorderseite eines vorderen Windelohres angebrachten Schlaufenmaterial. Mithin steht der männliche Teil dann nicht mehr als Teil eines mechanischen Verschlusses zwischen diesem männlichen Teil und dem an dem Windelverschlussband applizierten weiblichen Teil zur Verfügung.

Nicht gattungsgemäße Windelverschlussbänder offenbart die EP 0 832 631 A2, bei welcher, wie bei der DE 198 05 576 A1, ein Niederhalter dazu dient, ein Windelverschlussband betriebssicher um ein Windelohr gefaltet zu halten. In beiden Fällen stellt jedoch das Windelverschlussband lediglich einen Teil, nämlich den männlichen Teil, eines aus einem männlichen und einem weiblichen Teil bestehenden mechanischen Verschlusses bereit. Ebenfalls nicht gattungsgemäße Windelverschlussbänder offenbaren die EP 1 629 813 A1 und die US 2003/130644 A1, bei denen ein männlicher Teil eines aus einem männlichen und einem weiblichen Teil bestehenden mechanischen Verschlusses an dem Windelverschlussband befestigt ist und wenigstens zwei getrennte Trägerelemente für männliche Verschlusselemente umfasst. Auch die EP 0 894 448 A1 und die WO 98/27922 A1 offenbaren nicht gattungsgemäße Windelverschlussbänder, bei denen der männliche Teil eines aus einem männlichen und einem weiblichen Teil bestehenden mechanischen Verschlusses mit Klebstoff überzogen ist, um eine flexiblere Befestigungspositionen auf dem Windelkörper, insbesondere wenn die Windel eingerollt entsorgt werden soll, zu gewährleisten.

Entsprechende Windelverschlussbänder sind beispielsweise aus der DE 697 09 244 T2 bzw. der EP 0 813 851 B1 oder aber auch aus der DE 697 11 244 T2 bzw. der EP 0 941 728 A2 der EP 0 964 665 B1, der EP 1 000 598 A1, der US 5,019,073 und der US 5,176,670 bekannt. All diese Druckschriften offenbaren Windelverschlussbänder mit einer Befestigungsseite und mit einer Benutzerseite sowie mit einem von der Befestigungsseite zur Benutzerseite reichenden Trägerband, bei denen das Trägerband mit seiner Befestigungsseite permanent an einer Windel befestigt oder aber auch einstückig mit dieser ausgebildet sein kann und die Benutzerseite dazu dient, eine Windel nach dem Anlegen zu verschließen und ggf. zu öffnen und wieder zu verschließen. Hierzu ist an der Benutzerseite bei den Windelverschlussbändern dieser Druckschriften ein männlicher Teil eines aus einem männlichen und einem weiblichen Teil bestehenden mechanischen Verschlusses unlösbar befestigt, wobei mit diesem männlichen Teil der weibliche Teil des mechanischen Verschlusses verbunden ist und wobei an dem weiblichen Teil an der von dem männlichen Teil abweisenden Seite eine Klebstoffbeschichtung eines klebend wirksamen Verschlusses angeordnet ist. Es ist dabei nicht unbedingt zwingend, dass die Klebstoffbeschichtung unmittelbar an dem weiblichen Teil angeordnet ist, vielmehr können nach dem Stand der Technik hier auch Zwischenlagen vorgesehen sein.

Wird ein derartiges Windelverschlussband an einer Windel angebracht, so kann in einem ersten Schritt zunächst der weibliche Teil zum Verschließen an der Windel klebend mittels der Klebstoffbeschichtung befestigt werden. Durch den klebend wirksamen Verschluss und durch den mechanischen Verschluss ist dann die Windel verschlossen. Zum Öffnen und Wiederverschließen der Windel kann der mechanische Verschluss geöffnet werden, indem der männliche Teil ergriffen und von dem weiblichen Teil getrennt wird. Der weibliche Teil verbleibt mittels des klebend wirksamen Verschlusses dann an der Windel selbst und bildet ein Zielelement, auf welchem der männliche Teil zum Wiederverschließen der Windel jeweils wieder landen bzw. angebracht werden kann.

Insofern ist hierbei dann der weibliche Teil des mechanischen Verschlusses unlösbar an der Windel befestigt, während der männliche Teil des mechanischen Verschlusses entsprechend unlösbar an dem Trägerband des Windelverschlussbandes in dessen Benutzerbereich befestigt ist, welches seinerseits mit seinem Befestigungsbereich ebenfalls unlösbar an der Windel befestigt ist.

Hierbei ist es entsprechend der vorstehend benannten Druckschriften bekannt, den weiblichen Teil des mechanischen Verschlusses größer als den männlichen Teil auszubilden, so dass bei einem Wiederverschließen mehr Spielraum für eine Repositionierung des Verschlusses besteht. Dieses bedingt jedoch bis zum Positionieren des weiblichen Teils an der Windel Überstände des weiblichen Teils und mithin auch des hierauf angeordneten Klebstoffes, wodurch das Handling der Gesamtanordnung erschwert und insbesondere ein Umknicken der Überstände bedingt sein kann, welches dazu führt, dass die Klebstoffbeschichtung auf sich selber zur Anlage kommt und dieser Teil - wenn überhaupt - nur sehr schwer wieder entfaltet und für den Verschluss genutzt werden kann.

Es ist Aufgabe vorliegender Erfindung, ein Windelverschlussband, ein entsprechendes Bandmaterial sowie ein Verfahren zum Verschließen und Wiederverschließen einer Windel bereitzustellen, welche möglichst betriebssicher gehandhabt werden können. Als Lösung werden ein Windelverschlussband, ein entsprechendes Bandmaterial sowie ein Verfahren zum Verschließen und Wiederverschließen einer Windel mit den Merkmalen der Ansprüche 1, 11 und 12 vorgeschlagen.

Als Lösung wird einerseits ein Windelverschlussband mit einer Befestigungsseite und mit einer Benutzerseite sowie mit einem von der Befestigungsseite zur Benutzerseite reichenden Trägerband vorgeschlagen, wobei an der Benutzerseite des Trägerbandes ein erster Teil eines aus einem männlichen und einem weiblichen Teil bestehenden mechanischen Verschlusses unlösbar befestigt ist, wobei mit diesem ersten Teil der zweite Teil des mechanischen Verschlusses verbunden ist, wobei an dem zweiten Teil an der vom ersten Teil abweisenden Seite eine Klebstoffbeschichtung eines klebend wirksamen Verschlusses angeordnet ist und wobei sich das Windelverschlussband dadurch auszeichnet, dass der zweite Teil des mechanischen Verschlusses der männliche Teil ist und der erste Teil des mechanischen Verschlusses der weibliche Teil und der weibliche Teil des mechanischen Verschlusses größer als der männliche Teil ausgebildet ist.

Hierbei kann mithin das zweite Teil, also in diesem Falle der männliche Teil des mechanischen Verschlusses, sowie die an ihm angeordnete Klebstoffbeschichtung ein Target bilden, welches nach einem erstmaligen Verschließen an der Windel verbleibt und bei einem Wiederverschließen als Bestandteil des mechanischen Verschlusses hierfür zur Verfügung steht.

Darüber hinaus wird ein Verfahren zum Wiederverschließen einer Windel mit einem Windelkörper und mit einem entsprechenden Windelverschlussband vorgeschlagen, welches sich dadurch auszeichnet, dass der mechanische Verschluss geöffnet und der männliche Teil an den Windelkörper über die Klebstoffbeschichtung verbleibt und anschließend der weibliche Teil zum Wiederverschließen wieder an den männlichen Teil appliziert wird.

Insofern kehren sich die vorstehend beschriebenen Lösungen von der Tradition, den weiblichen Teil eines mechanischen Verschlusses an dem Windelkörper beispielsweise durch ein Frontal-Tape oder aber durch ein Non-Woven auf der gesamten Windelohrseite sowie das Hakenmaterial am Windelverschlussband vorzusehen, welche sich letztlich auch in dem eingangs genannten Stand der Technik dementsprechend manifestiert, da auch dort das Hakenmaterial am Windelverschlussband auf die Windel zuweisend angeordnet ist, ab und ordnen das Hakenmaterial derart an, dass es im verschlossenen Zustand von der Windel weg weist.

Diese Anordnung hat unter anderem bei geeigneter Ausgestaltung des Windelverschlussbands den Vorteil, dass letztlich preisgünstigere männliche bzw. weibliche Teile des mechanischen Verschlusses zur Anwendung kommen können, da naturgemäß die männlichen Teile in der Regel wesentlich eigensteifer als die weiblichen Teile sind. Wird nunmehr das Windelverschlussband an der Windel zum Verschließen appliziert, so drückt ein Benutzer bei der vorliegenden Lösung unmittelbar den weicheren weiblichen Teil in den männlichen Teil hinein, was zu einer erheblich besseren Verbindung der beiden Teile des mechanischen Verschlusses führt, als dieses gelingen kann, wenn der Benutzer auf den verhältnismäßig steifen männlichen Teil drückt, da weniger punktuelle Lasten sondern eher flächige Lasten von dem verhältnismäßig eigensteifen männlichen Teil aufgenommen und an die Verbindung zum weiblichen Teil weitergegeben werden. Die durch die vorliegende Lösung bedingte, verbesserte Verbindung zwischen den beiden Teilen des mechanischen Verschlusses kann beispielsweise dazu benutzt werden, diese kleiner und mithin kostengünstiger auszubilden. Ebenso ist es denkbar, preisgünstigere und somit von Natur aus weniger gut eine Verbindung eingehende Teile des mechanischen Verschlusses miteinander zu kombinieren.

In Verbindung mit der oben genannten Lösung wird weiter ein Windelverschlussband mit einer Befestigungsseite und mit einer Benutzerseite sowie mit einem von der Befestigungsseite zur Benutzerseite reichenden Trägerband vorgeschlagen, wobei an der Benutzerseite des Trägerbandes ein erster Teil eines aus einem männlichen und einem weiblichen Teil bestehenden mechanischen Verschlusses unlösbar befestigt ist, wobei mit diesem ersten Teil der zweite Teil des mechanischen Verschlusses verbunden ist, wobei an dem zweiten Teil an der vom ersten Teil abweisenden Seite eine Klebstoffbeschichtung eines klebend wirksamen Verschlusses angeordnet ist und wobei sich das Windelverschlussband dadurch auszeichnet, dass der männliche Teil des mechanischen Verschlusses wenigstens zwei getrennte Trägerelemente für männliche Verschlusselemente umfasst. Unabhängig von den Merkmalen der oben genannten Lösungen, insbesondere aber auch im Zusammenspiel mit diesen Lösungen, kann hierdurch die Festigkeit der Verbindung der beiden Teile des mechanischen Verschlusses, insbesondere bei einem Verschließen der Windel, erhöht werden, so dass dementsprechend auch die Betriebssicherheit erhöht werden kann. Hierbei wird auch in Abgrenzung zu der WO 2005/074852 A1 durch das Target, welches den zweiten Teil des mechanischen Verschlusses umfasst und nach einem erstmaligen Verschließen der Windel an der Windel zum Wiederverschließen verbleibt, ein entsprechend stabilerer, insbesondere von der Windeloberfläche an sich unabhängiger, mechanischer Verschluss bereitgestellt. Letzteres gilt unabhängig davon, welcher der beiden Teile des mechanischen Verschlusses nun männlich und welcher weiblich ausgestaltet ist, wobei die Vorteile entsprechend kumulieren, wenn der zweite Teil männlich ausgebildet ist. Dementsprechend ist es wie bei der erst genannten Lösung möglich, bei geeigneter Ausgestaltung des Windelverschlussbands preisgünstigere männliche bzw. weibliche Teile des mechanischen Verschlusses zu nutzen, da durch das Vorhandensein von getrennten Trägerelementen und die hierdurch erhöhte Flexibilität des männlichen Teils des mechanischen Verschlusses bzw. die hierdurch erhöhte Zahl an Randbereichen, welche letztlich dementsprechend mehr männliche Verschlusselemente am Rand eines Trägerelements zur Verfügung stellen, die Bindungsstärke der entsprechenden Verbindung gegenüber herkömmlichen Anordnungen erhöht ist.

In diesem Zusammenhang sei betont, dass männliche Verschlusselemente, wie Haken, Pilzköpfe oder ähnliches, in der Regel in Trägerelementen gelagert sind, die sicherstellen, dass die männlichen Verschlusselemente ausreichend steif aufstehen, um so gezielt mit korrespondierenden weiblichen Verschlusselementen wechselwirken zu können. Dementsprechend sind in der Regel nicht nur die männlichen Verschlusselemente sondern auch die Trägerelemente verhältnismäßig steif ausgebildet, insbesondere im Verhältnis zu weiblichen Teilen derartiger mechanischer Verschlüsse. Aus Gründen der Fertigung sind die männlichen Verschlusselemente häufig sogar einstückig mit ihren Trägerelementen ausgebildet. Umfasst der männliche Teil des mechanischen Verschlusses mithin wenigstens zwei getrennte Trägerelemente, so wird die mit den Trägerelementen verbundene Steifigkeit aufgebrochen, so dass die entsprechenden männlichen Verschlusselemente wesentlich inniger mit den korrespondierendem weiblichen Teil des mechanischen Verschlusses in Wechselwirkung treten können.

Die Trägerelemente können beispielsweise durch das Trägerband oder durch sonstige Maßnahmen, wie beispielsweise eine Folie, eine ausreichend stabile Klebstoffschicht oder eine sonstige tragfähige Lage verbunden sein, die biegsamer als die Trägerelemente sind.

Darüber hinaus kann ein Windelverschlussband mit einer Befestigungsseite und mit einer Benutzerseite sowie mit einem von der Befestigungsseite zur Benutzerseite reichenden Trägerband vorgesehen sein, wobei an der Benutzerseite des Trägerbandes ein erster Teil eines aus einem männlichen und einem weiblichen Teil bestehenden mechanischen Verschlusses unlösbar befestigt ist, wobei mit diesem ersten Teil der zweite Teil des mechanischen Verschlusses verbunden ist, wobei an dem zweiten Teil an der vom ersten Teil abweisenden Seite eine Klebstoffbeschichtung eines klebend wirksamen Verschlusses angeordnet ist und wobei sich das Windelverschlussband dadurch auszeichnet, dass zusätzlich zu dem mechanischen Verschluss ein klebend zwischen männlichem und weiblichem Teil wirksamer Zusatzverschluss vorgesehen ist. Ein derartiger Zusatzverschluss kann insbesondere dazu genutzt werden, die Gefahr eines Aufspringens des mechanischen Verschlusses zu reduzieren, wodurch sich die Betriebssicherheit weiter erhöhen lässt bzw. sich preisgünstigere und/oder auch weniger aggressive Materialien für den mechanischen Verschluss, insbesondere für dessen männlichen Teil, verwenden lassen. Weniger aggressive Materialien haben ggf. auch den Vorteil einer angenehmeren Haptik, insbesondere wenn der männliche Teil des mechanischen Verschlusses an die Außenseite einer Windel über die an der von dem ersten Teil abweisenden Seite eine Klebstoffbeschichtung appliziert ist. Durch die Kombination von mechanischem Verschluss und klebend wirksamen Zusatzverschluss können insbesondere Scherkräfte von dem mechanischen Verschluss und Aufspringkräfte, also senkrecht zur Ebene des Verschlusses bzw. senkrecht zu den Scherkräften wirksame Kräfte, von dem klebend wirksamen Zusatzverschluss weitgehend übernommen werden. Der Zusatzverschluss ist beispielsweise derart ausgebildet, dass er unmittelbar zwischen dem männlichen und dem weiblichen Teil wirksam ist, indem beispielsweise eine Klebstoffschicht auf einem der Teile aufgebracht wird, die dann mit dem anderen der Teile dementsprechend in Wechselwirkung treten kann. Andererseits kann der Zusatzverschluss auch indirekt verschließend zwischen dem männlichen und dem weiblichen Teil klebend wirksam werden, indem eine mit einem der beiden Teile fest verbundene Baugruppe, wie beispielsweise ein Trägerband oder eine Trägerschicht, eine Komponente des Zusatzverschlusses trägt und mit dem anderen er beiden Teile entsprechend klebend wechselwirkt, um so die beiden Teile neben der mechanischen Verbindung klebend zu verbinden.

Der Klebstoff des klebend zwischen männlichem und weiblichen Teil wirksamen Zusatzverschlusses ist hierbei derart eingestellt, dass der männlichen und der weiblichen Teil des mechanischen Verschlusses unter normalen Betriebsbedingungen bzw. Nutzungsbedingen von einem Nutzer in Abweichung von unlösbar befestigten Baugruppen ohne weiteres geöffnet und wieder verschlossen werden kann.

Räumlich neben dem männlichen Teil des mechanischen Verschlusses kann eine Klebstoffbeschichtung eines klebend wirksamen Zusatzverschlusses vorgesehen sein, wodurch die vorstehend genannte Lösung baulich besonders einfach umgesetzt werden kann. Insbesondere kann ggf. eine ohnehin zum Verbinden des männlichen Teils mit anderen Schichten oder Lagen, beispielsweise mit dem Trägerband oder mit der Klebstoffbeschichtung des klebend wirksamen Verschlusses, vorhandene Klebstoffbeschichtung hierfür genutzt werden. Ebenso ist es denkbar, sogar die Klebstoffbeschichtung des klebend wirksamen Verschlusses dementsprechend als Klebstoffbeschichtung des Zusatzverschlusses zu nutzen.

Die vorgenannten Lösungen, die Anordnung der Klebstoffbeschichtung an dem männlichen Teil des mechanischen Verschlusses, der Einsatz von getrennten Trägerelementen für die männlichen Verschlusselemente und der klebend wirksame Zusatzverschluss, gehen allesamt von der gemeinsamen erfinderischen Grundidee aus, dass die eingangs genannte Aufgabe der erhöhten Betriebssicherheit dadurch gelöst wird, dass dem Benutzer zum Verschließen des gattungsgemäßen mechanischen Verschlusses eine weichere Oberfläche angeboten wird, so dass er mit seinem Händen bzw. Fingern den mechanischen Verschluss betriebssicher fester verschließen kann, als dieses bei sehr eigensteifen Baugruppen möglich ist. So kann ein Benutzer den außen liegenden weicheren weiblichen Teil eines mechanischen Verschlusses inniger mit dem männlichen Teil verbinden, wenn er mit einem Finger den weiblichen Teil lokal verstärkt in den männlichen Teil hineintreibt. Ebenso kann ein Benutzer einen weicheren und flexibleren männlichen Teil eines mechanischen Verschlusses, sei es weicher aufgrund von getrennten Trägerelementen oder sei es weicher aufgrund der Verwendung kleinerer oder weicherer durch den klebend wirksamen Zusatzverschluss ermöglichter Trägerelemente, besser mit dem weiblichen Teil verbinden, wobei diese Vorteile bei Verwendung zweier oder aller dieser Lösungsansätze in Kombination entsprechend kumuliert auftreten.

Während bei der Lösung, bei welcher die Klebstoffbeschichtung an dem männlichen Teil vorgesehen ist, die Klebstoffbeschichtung und der männliche Teil das Target bilden, welches nach einem erstmaligen Verschließen an der Windel verbleibt und bei einem Wiederverschließen als Bestandteil des mechanischen Verschlusses für ein Wiederverschließen zur Verfügung steht, kann das Target bei den Lösungen, bei welchen der getrennte Trägerelemente für die männlichen Verschlusselemente und/oder der klebend wirksame Zusatzverschluss zum Einsatz kommen, auch statt des männlichen Teils auch den weiblichen Teil des mechanischen Verschlusses aufweisen, wobei letztlich insbesondere bei einer Kombinationen dieser Lösung mit der Lösung, bei welcher die Klebstoffbeschichtung an dem männlichen Teil vorgesehen ist, das Target auch den männlichen Teil des mechanischen Verschlusses umfassen kann. Es versteht sich, dass das Target neben einem teil des mechanischen Verschlusses und der Klebstoffbeschichtung noch weitere Bestandteile, wie Trägerfolien, farbige Schichten, Beschichtungen oder ähnliches, umfassen kann.

Ohne Weiteres kann bei den vorliegenden Lösungen, insbesondere bei der Lösung, bei welcher der männlichen Teil des mechanischen Verschlusses als zweiten Teil genutzt und mit der Klebstoffbeschichtung des klebend wirksamen Verschlusses versehen ist, der weibliche Teil des mechanischen Verschlusses größer als der männliche Teil ausgebildet werden, so dass die Möglichkeit eines Repositionierens des weiblichen Teils im Bezug auf den männlichen Teil in verhältnismäßig großen Grenzen möglich ist. Dadurch, dass auf der Rückseite des männlichen Teils die Klebstoffschicht vorgesehen ist, ist diese zum einen verhältnismäßig klein, so dass schon aus diesem Grunde die Wahrscheinlichkeit, dass ein Umknicken stattfindet und die Klebstoffschicht auf sich selbst zum Ruhen kommt, äußerst gering ist. Diese Wahrscheinlichkeit reduziert sich noch dadurch, wenn der männliche Teil naturgemäß verhältnismäßig eigensteif ist. Der flexiblere weibliche Teil, der größer als der männliche Teil ausgebildet ist, weist hingegen keine freie Klebstoffbeschichtung auf, so dass hier ein etwaiges Umknicken unkritisch ist.

Bei Windelverschlussbändern lässt sich in der Regel eine Längs- und eine Querrichtung definieren, wobei die Längsrichtung von der Befestigungsseite zur Benutzerseite weist und letztlich auch durch den Überstand definiert ist, welchen das Windelverschlussband von der Windel ausgehend bis zu seinem Verschlusselement überbrückt. Die Querrichtung liegt senkrecht zu dieser Längsrichtung und senkrecht zur Dicke des Windelverschlussbandes, also beispielsweise senkrecht zur Dicke des Trägerbandes. Bei der Herstellung derartiger Windelverschlussbänder verläuft die Querrichtung häufig in Maschinenrichtung, während die Längsrichtung des Windelverschlussbands dementsprechend quer zur Maschinenrichtung vorgesehen ist. Insofern kann der weibliche Teil des mechanischen Verschlusses sowohl in Längsrichtung als auch in Querrichtung größer als der männliche Teil ausgebildet sein. In vorliegendem Zusammenhang bezeichnet der Begriff "größer" die von dem jeweiligen Teil zur Wechselwirkung mit dem anderen Teil zur Verfügung stehende Fläche. Insofern ist es rein theoretisch auch denkbar, dass der männliche Teil in bestimmten Bereichen einen gewissen kleineren Überstand aufweist, was letztlich, insbesondere da in der Regel männliche Teile eines mechanischen Verschlusses kostenintensiver als die weiblichen Teile sind, in der Regel wenig Sinn macht.

Darüber hinaus hat sich herausgestellt, dass bei einem Repositionieren des weiblichen Teils auf den männlichen Teil Abweichungen in Querrichtung des Windelverschlussbands nur sehr selten korrigiert werden müssen, da derartige Abweichungen beim Verschließen einer Windel in großen Grenzen unkritisch sind. Anders sieht dieses in Längsrichtung des Windelverschlussbandes aus, da häufig schon nach kurzer Zeit die Windel an die genaue Körperpassform des jeweiligen Trägers besser angepasst ist, so dass bei einem Repositionieren die Windelverschlusslänge verkürzt werden sollte, damit die Windel nach wie vor straff und sicher sitzt. Eine derartige Verkürzungen der Windelverschlusslänge kann mit der vorliegend beschriebenen Lösung ohne Weiteres ermöglicht werden, indem der weibliche Teil in Richtung der Befestigungsseite länger als der männliche Teil ausgebildet ist. Entsprechend dieser Länge besteht bei einem Repositionieren Spielraum, den weiblichen Teil straffer anzuziehen und mithin näher der Befestigungsseite mit dem männlichen Teil zu verbinden, so dass die Windelverschlusslänge, welche durch dieses Windelverschlussband bereitgestellt wird, entsprechend verkürzt ist. Insbesondere eine derartige Ausgestaltung ist bei den eingangs genannten Windelverschlussbändern nach dem Stand der Technik nur äußerst kompliziert bzw. aufwändig zu lösen, da ein derartiger Überstand des weiblichen Teils zu einer entsprechenden Verlängerung bzw. Vergrößerung des Windelverschlussbands und den hiermit verbundenen Instabilitäten führt. Eine entsprechende Vergrößerung des männlichen Verschlussteils führt hingegen zu einer erheblichen Kostensteigerung, da in der Regel die männlichen Verschlussteile wesentlich kostenintensiver als die weiblichen Verschlussteile sind.

Während bereits ein sehr kleiner Überstand des weiblichen Teils in Richtung auf die Befestigungsseite ausreicht, um dementsprechend die Repositionierbarkeit zu erhöhen, ist es insbesondere von Vorteil, wenn der weibliche Teil des mechanischen Verschlusses zumindest über die halbe Länge des Trägerbands vorgesehen ist. Auf diese Weise kann sehr einfach genügend Freiraum für ein Verkürzen der Windelverschlusslänge bereitgestellt werden.

In diesem Zusammenhang sei betont, dass der weibliche Teil des mechanischen Verschlusses auch über die gesamte Länge des Trägerbandes ausgebildet sein kann, wobei ggf. ein Griffelement, welches ein Ergreifen des Benutzerendes des Trägerbandes an der Benutzerseite erleichtern soll, hier unberücksichtigt bleibt und wobei ggf. ein Teil des weiblichen Teils von einem Windelohr oder einem Windelkörper an der Befestigungsseite überdeckt wird, um dort das Windelverschlussband permanent an der Windel zu befestigen, ohne dass von dem Grundgedanken, möglichst viel der Länge des Trägerbands mit dem weiblichen Teil des mechanischen Verschlusses zu versehen, abgewichen wird.

Eine baulich besonders einfache Umsetzung des Windelverschlussbandes kann gewährleistet werden, wenn das Trägerband den weiblichen Teil des mechanischen Verschlusses bildet. Beispielsweise kann das Trägerband hierzu ein ausreichend stabiles Non-Woven oder Gewirke bzw. ein ausreichend stabiles anderes textiles Material sein, in welches der männliche Teil des mechanischen Verschlusses, beispielsweise ein Hakenmaterial, eingreifen kann.

In einer alternativen Umsetzung kann das Trägerband den weiblichen Teil des mechanischen Verschlusses tragen, indem dieser beispielsweise auf das Trägerband aufkaschiert ist. Zum Beispiel kann dieses ein aufkaschiertes Non-Woven oder eine sonstige Schlaufen oder ausreichende weiche Hinterschneidungen bildende Materiallage, wie beispielsweise eine Lage aus in eine Folie, ggf. auch in eine elastische Folie, extrudierten Stapelfasern, sein.

Das Trägerband kann hierbei elastisch ausgebildet sein und insbesondere beispielsweise auch aus einem zumindest in eine Richtung elastischen Non-Woven, elastischen Gewirke oder einem sonstigen elastischen Material, welches mit einem textilen Material kaschiert ist, bestehen. Kumulativ oder alternativ hierzu kann das Trägerband elastische Bereiche aufweisen, welche beispielsweise durch eingesetzte Elastomere gebildet sind. Ebenso können derartige elastische Bereiche durch einen gezielten Streckvorgang oder sonstige gezielte Materialschwächungen oder Ähnliches bereitgestellt werden, wie dieses aus dem Stand der Technik hinlänglich bekannt ist. Wie bereits vorstehend angedeutet, können entsprechende Trägerbänder einlagig aber auch mehrlagig ausgebildet sein, so dass insbesondere auch unelastische mit elastischen Lagen verbunden sein können, wobei dann in ausgewählten elastischen Bereichen die unelastischen Lagen entsprechend geschwächt oder zerteilt werden, um eine Elastizität zu ermöglichen. Eine gewisse Elastizität des Trägerbands auch in dem Bereich, in dem eine Wechselwirkung mit dem männlichen Teil des mechanischen Verschlusses vorgesehen ist, kann den Vorteil haben, dass dort erhöhte Scherkräfte auftreten, die eine bessere Verbindung zwischen männlichem Teil und dem den weiblichen Teil des mechanischen Verschlusses bildenden Trägerbandes bedingen. Dementsprechend kann das Trägerband auch mehrlagig oder mehrstückig ausgebildet sein. Ebenso ist es denkbar, das Trägerband einstückig mit der Windel auszubilden.

Ein mechanischer Verschluss weist einen Teil mit etwas härteren und/oder spitzeren Elementen auf, welche in etwas weichere und/oder rundere Elemente eindringen und auf diese Weise den Verschluss schließen. Hierbei werden die härteren Teil üblicher Weise als männliche Teile und die weicheren Teile üblicher Weise als weibliche Teile bezeichnet. Häufig weisen die männlichen Teile Haken, Pilzköpfe oder sonstige Hinterschneidungen auf, wobei Materialien mit derartigen Hinterschneidungen üblicher Weise zusammenfassend als Hakenmaterialien bezeichnet werden, auch wenn beispielsweise Pilzköpfe oder Ähnliches nicht im engeren Sinne nicht als Haken zu verstehen sein sollten. Häufig weisen die weiblichen Teile derartiger mechanischen Verschlüsse einen textilen Charakter auf und sind gewebt und gewirkt, wobei dann die entsprechenden männlichen Gegenstücke hinter die jeweiligen Fäden, Fasern oder Filamente greifen, um den Verschluss zu schließen. Naturgemäß können derartige Fäden, Fasern oder Filamente einem Druck leicht nachgeben und ausweichen, so dass auf diese Weise die Haken einen entsprechenden Verschluss schaffen können. Hierbei ist es jedoch nicht zwingend notwendig, dass die weiblichen Teile Schlaufen oder Schleifen, die im klassischen Sinne geschlossen sind, aufweisen müssen. Vielmehr reichen auch Fasern- oder Filamentteil-oder -endstücke aus, um ein ausreichendes Einhaken bzw. eine ausreichende Verbindung mit den männlichen Teilen zu gewährleisten, so dass auch insbesondere Non-Woven-Materialien aber auch sonstige, verschließend mit den männlichen Teilen wechselwirkende Materialien als weibliche Teile genutzt werden können. Insoweit die männlichen Teile Hakenmaterialien umfassen, wird üblicher Weise das hiermit korrespondierende Material als Schlaufenmaterial bezeichnet, solange diese Fäden, Fasern oder Filamente aufweist, welche für eine Wechselwirkung mit den Haken des Hakenmaterials geeignet sind.

Vorzugsweise ist der klebend wirksame Verschluss mittels eines Release-Tapes abgedeckt, welches auf seiner dem klebend wirksamen Verschluss zugewandten Seite klebstoffabweisend und auf seiner dem klebend wirksamen Verschluss abgewandten Seite klebend ausgebildet ist. Ein derartiges Release kann, wenn das entsprechende Windelverschlussband an der Windel befestigungsseitig angebracht wird, dementsprechend an der Windel angebracht werden oder aber bereits vorab mit dem Windelverschlussband an entsprechender Stelle verbunden sein. Mit seiner klebstoffabweisenden Seite kann dann die Klebstoffschicht des klebend wirksamen Verschlusses durch das Release-Tape dementsprechend positioniert verbleiben, bis ein Benutzer diese klebend wirksame Verbindung löst und das Windelverschlussband benutzerseitig einschließlich des männlichen Teils des mechanischen Verschlusses und der an dem männlichen Teil vorgesehenen Klebstoffschicht ergreift, um die Windel zu verschließen, indem dieser männliche Teil mittels der von ihm getragenen Klebstoffschicht auf dem Windelkörper bzw. auf dem Backsheet einer Windel in geeigneter Position positioniert wird. Hierbei gewährleistet die klebstoffabweisende Beschichtung des Release-Tapes, dass der männliche Teil einschließlich seiner Klebstoffbeschichtung ohne Weiteres von dem Release-Tape abgezogen werden kann. Auf diese Weise bleibt insbesondere auch die Klebstoffbeschichtung im Wesentlichen unbeeinträchtigt, so dass ein sicherer klebender Sitz auf dem Windelkörper bzw. auf Backsheet der Windel gewährleistet ist.

Insbesondere wenn das Windelverschlussband als Bandmaterial auf einer Rolle ausgeliefert und erst durch Trennen der Rollen rechtwinklig zur Rollrichtung, diese entspricht in der Regel der Maschinenrichtung, in einzelne Windelverschlussbänder getrennt wird, kann die Anordnung aus Trägerband, Release-Tape und mechanischen und klebend wirksamen Verschlüssen derart gestreckt sein, dass eine klebende Seite des Trägerbands und eine klebende Seite des Release-Tapes nach oben weisen und das Release-Tape noch auf der Benutzerseite des Windelverschlussbandes ruht, wobei diese Anordnung dann mit ihren klebenden Seiten um den Rand einer Windel herum gefaltet wird, so dass der Rand der Windel zu einem von der Befestigungsseite des Trägerbandes und zum anderen von dem Release-Tape umgriffen wird. Mit dem Abziehen des männlichen Teils einschließlich seiner Klebstoffbeschichtung ergibt sich dann das befestigungsseitige Ende des Trägerbandes aus Trägerband und Release-Tape, welche beide permanent mit ihren Klebstoffbeschichtungen an der Windel befestigt sind, und das benutzerseitige Ende des Trägerbandes, welche durch das Benutzerende des Trägerbandes, dem mechanischen Verschluss und die Klebstoffbeschichtung des klebend wirksamen Verschlusses gebildet wird.

Insbesondere wenn das Windelverschlussband einen mechanischen Verschluss aufweist, an dessen männlichem Teil an seiner von dem weiblichen Teil abweisenden Seite die Klebstoffbeschichtung des klebend wirksamen Verschlusses angeordnet ist und mithin das Trägerband den weiblichen Teil des mechanischen Verschlusses trägt oder bildet, und wenn ein Release-Tape vorgesehen ist, kann es von Vorteil sein, dass auch das Release-Tape auf seiner der Windel bzw. dem Windelkörper abgewandten Seite eine textil ausgestaltete Oberfläche, wie beispielsweise eine Gewirkelage, eine Gewebelage oder eine Non-Woven-Lage, aufweist oder sogar zur Gänze aus einem textilen Material, wie einem Gewirke, einem Gewebe oder einem Non-Woven, gebildet ist. Auf diese Weise ist eine Repositionierung des mechanischen Verschlusses auch im Bereich des Release-Tapes ohne Weiteres möglich. Bei einer derartigen Ausgestaltung ist ggf. die textil ausgestaltete Oberfläche bzw. das textile Material auf seiner der Windel bzw. dem Windelkörper abgewandten Seite in geeigneter Weise klebstoffabweisend zu beschichten, was beispielsweise durch das Auftragen oder anderweitige Aufbringen von Silikon oder anderen klebstoffabweisenden Stoffen geschehen kann, so dass die Klebstoffbeschichtung des klebend wirksamen Verschlusses einfach von dem Release-Tape abgezogen werden kann, ohne irgend welche textilen Elemente, wie Fäden oder Fasern auszureißen oder sonstwie kontaminiert und in der Klebfähigkeit erheblich beeinträchtigt zu werden.

Ebenso versteht es sich, dass ggf. auch eine dem Hakenmaterial zugewandte Seite des Windelkörpers bzw. eine dem männlichen Teil zugewandte Seite des Windelkörpers kumulativ oder alternativ zu dem Release-Tape für eine Repositionierung genutzt werden kann, wenn die Oberfläche des Windelkörpers für einen Verschluss mit dem männlichen Teil geeignet ist. In diesem Zusammenhang sei betont, dass genau in letzterem Fall der weibliche Teil des mechanischen Verschlusses, welcher an dem Trägerband befestigungsseitig unlösbar befestigt ist, bzw. das Trägerband selbst oder auch das Release-Tape, falls ein derartiges zur Anwendung kommt, aus dem Material gefertigt sein können oder das Material tragen können, das die dem männlichen Teil zugewandte Seite des Windelkörpers bildet. Selbst wenn letzteres nicht umgesetzt wird, kann es insbesondere aus Kostengründen aber auch aus Gründen der einfacheren Konfektionierbarkeit von Vorteil sein, das Trägerband und das Release-Tape aus demselben Material zu fertigen oder zumindest an der jeweils dem männlichen Teil zugewandten Seite mit identischem, den weiblichen Teil des mechanischen Verschlusses bereitstellendem Material zu kaschieren.

Insofern wird die eingangs erläuterte Aufgabe auch durch eine Windel bzw. durch ein Windelohr mit entsprechend ausgestalteten Windelverschlussbändern gelöst, insbesondere auch wenn das Trägerband einstückig mit der Windel bzw. mit dem Windelohr ausgestaltet ist.

Vorzugsweise werden die Windelverschlussbänder aus einem Bandmaterial mit einer Längserstreckungsrichtung gefertigt, indem dieses Bandmaterial quer zur Längserstreckungsrichtung zu Einzelnutzen in Form von Windelverschlussbändern getrennt wird. Dementsprechend liegt dann die Längsrichtung des auf diese Weise bereitgestellten Windelverschlussbandes senkrecht zur Längserstreckungsrichtung des Bandmaterials, welche dementsprechend ihrerseits einer Maschinenrichtung entspricht, in welcher das Bandmaterial eine entsprechende Fertigungsmaschine verlässt. Je nach konkreter Umsetzung kann das Bandmaterial auch mehrnutzig, insbesondere doppelnutzig, vorbereitet sein, so dass in Quererstreckungsrichtung zwei oder mehr Bereiche des Bandmaterials zu Windelverschlussbändern abgetrennt werden können. Insofern kann ggf. auch ein Trennen in Längserstreckungsrichtung erfolgen, was - je nach konkreter Umsetzung - vor oder dem Trennen quer zur Längserstreckungsrichtung des Bandmaterials oder während des Trennens quer zur Längserstreckungsrichtung des Bandmaterials erfolgen kann.

Zum Transport derartigen Bandmaterials wird dieses vorzugsweise aufgewickelt, was in Form von Rollen, bei denen Wicklung auf Wicklung Lage über Lage gleichförmig und ohne axialen Versatz übereinander gelegt wird, oder in Form von Spulen, bei denen einzelne, wenn auch nicht zwingend alle, Wicklungen mit einem axialen Versatz übereinander gelegt werden, was auch als Kreuzwicklung definiert ist. Überraschender Weise hat sich herausgestellt, dass durch eine derartige Kreuzwicklung die Betriebssicherheit an der Windelmaschine insbesondere beim Durchtrennen des Bandmaterials zu Einzelnutzen und dem hiernach folgenden Applizieren derselben als Windelverschlussbänder gegenüber anderen Auslieferungsformen, insbesondere gegenüber Rollen, erheblich erhöht ist. Hierbei wird davon ausgegangen, dass dieses daran liegt, dass die vorstehend erläuterten Windelverschlussbänder aufgrund ihres Aufbaus aus Trägerband, mechanischem Verschluss und Klebstoffbeschichtung sowie ggf. dem Release-Tape eine verhältnismäßig große Dicke aufweisen, was beim Aufwickeln und beim Abwickeln zu Verspannungen innerhalb dieses Aufbaus führt. entsprechend der Beziehung zwischen Umfang U und Radius r:

$$U = 2\pi r$$

folgt bei einem Abstand a zwischen zwei auf einem inneren Umfang $U_i$ und einem äußeren Umfang $U_a$ aufgewickelte Materiallagen, beispielsweise dem Trägerband einerseits und dem Trägermaterial für das Hakenmaterial andererseits oder aber beispielsweise zwischen dem Trägerband einerseits und einem mit Klebstoff beschichteten Flicken eines weiblichen Teil eines mechanischen Verschlusses, der mit dem männlichen Teil des mechanischen Verschlusses verbundenen ist, dessen Trägermaterial wiederrum mit dem Trägerband verbunden ist, andererseits für zu Verspannungen führenden Wegunterschied $\Delta$:

$$\Delta = U_a - U_i \; ; \; \text{mit } U_a = 2\pi r_a \wedge U_i = 2\pi r_i \wedge r_i + a = r_a$$

$$\Rightarrow \Delta = 2\pi r_a - 2\pi r_i = 2\pi a$$

ein linearer Zusammenhang zu dem Abstand a, der insbesondere unabhängig von dem Radius ist, so dass eigentlich die Verspannungen bei Rollen geringer ausfallen sollten, da je Lage weniger Wicklungen aufgewickelt werden. Offensichtlich ist jedoch unerwarteter Weise genau das Gegenteil der Fall, so dass davon ausgegangen wird, dass durch eine Kreuzwicklungen entsprechende durch den Wegunterschied $\Delta$ bedingte Verspannungen besser abgebaut werden können, indem das Material seitlich ausweichen kann.

Dementsprechend löst unabhängig von den übrigen Merkmalen, die ein Windelverschlussband noch aufweisen kann, die eingangs gestellte Aufgabe eine Kreuzwicklung eines Bandmaterials mit einer Längserstreckungsrichtung, welches durch Trennen quer zur Längserstreckungsrichtung, und gegebenenfalls durch Trennen in Längserstreckungsrichtung, zu Einzelnutzen in Form von Windelverschlussbändern mit einer Befestigungsseite und mit einer Benutzerseite sowie mit einem von der Befestigungsseite zur Benutzerseite reichenden Trägerband, wobei an der Benutzerseite des Trägerbandes ein erster Teil eines aus einem männlichen und einem weiblichen Teil bestehenden mechanischen Verschlusses unlösbar befestigt ist, wobei mit diesem ersten Teil der zweite Teil des mechanischen Verschlusses verbunden ist und wobei an dem zweiten Teil an der von dem ersten Teil abweisenden Seite eine Klebstoffbeschichtung eines klebend wirksamen Verschlusses angeordnet ist, bevorzugt ohne Verschnitt, aufteilbar ist. Obgleich durch den beide Teile umfassenden mechanischen Verschluss eine große Dicke des Bandmaterials vorliegt, können durch die Kreuzwicklung Materialspannungen, die zu Verwerfungen führen und den nachfolgenden Prozess beeinträchtigen können, minimiert werden.

Hierbei versteht es sich, dass, wenn das Trägerband den weiblichen Teil des mechanischen Verschlusses als ersten Teil bildet und der zweite Teil des mechanischen Verschlusses an seiner von dem Trägerband wegweisen Seite die Klebstoffbeschichtung des klebend wirksamen Verschlusses aufweist, wie dieses in Anspruch 2 beansprucht ist, derartige Verspannungen weiter minimiert werden können, da die Dicke des Windelverschlussbandes und mithin des aufgewickelten Bandmaterials weiter minimiert werden kann. Diese bevorzugte Ausgestaltung ermöglicht nämlich auf ein separates weibliches Teil des mechanischen Verschlusses, welches ansonsten die Dicke weiter erhöht, zu verzichten. Ebenso ermöglicht die Verwendung von getrennten Trägerelementen für die männlichen Verschlusselementen und/oder die Verwendung eines zu dem mechanischen Verschluss ergänzend klebend wirksamen Zusatzverschlusses den Einsatz männlicher Teile des mechanischen Verschlusses, die eine sehr geringe Dicke aufweisen und mithin zunächst nicht so gute Verbindungen eingehen, was wiederrum durch die getrennten Trägerelemente und des klebend wirksamen Zusatzverschluss zumindest teilweise kompensiert werden kann und wegen der entsprechend geringeren Dicke wiederrum mögliche Verspannungen minimiert.

Wie bereits vorstehend erläutert, kann das Release-Tape, insbesondere wenn es eine textile, der Klebstoffbeschichtung des klebend wirksamen Verschlusses zugewandte Oberfläche aufweist, mit einer klebstoffabweisenden Schicht zu beschichten oder sonstwie klebstoffabweisend auszugestalten, was beispielsweise durch das Auftragen oder anderweitige Aufbringen von Silikon oder anderen klebstoffabweisenden Stoffen geschehen kann und letztlich auch bei Release-Tapes, die keinen textilen Charakter aufweisen, je nach Wahl des Klebstoffes entsprechend vorteilhaft sein kann. Hierdurch lässt sich eine Korruption des Klebstoffes weitgehend vermeiden, wenn dieser von dem Release-Tape abgezogen, an der Windel angebracht und gut dort kleben soll. Aus demselben Grund kann es von Vorteil sein, die Rückseite des Trägerbandes, also die dem mechanischen Verschluss und der Klebstoffbeschichtung des klebend wirksamen Verschlusses und ggf. einem Release-Tape abgewandte Seite des Trägerbandes, dementsprechend klebstoffabweisend auszubilden. Auch dieses kann beispielsweise durch das Auftragen einer geeigneten Beschichtung, was beispielsweise durch das Auftragen oder anderweitige Aufbringen von Silikon oder anderen klebstoffabweisenden Stoffen geschehen kann. Auf diese Weise kann die Gefahr reduziert werden, dass der Klebstoff der Klebstoffbeschichtung des klebend wirksamen Verschlusses, falls kein diese Klebstoffbeschichtung kaschierendes Release-Tape zur Anwendung kommt, oder aber eine Klebstoffbeschichtung des Release-Tapes bzw. eine sonstige befestigungsseitige Klebstoffbeschichtung, die zur dauerhaften Befestigung des Windelverschlussbands an einer Windel dient, beeinträchtigt wird, wenn ein entsprechendes Bandmaterial, aus welchem dann die Windelverschlussbänder gefertigt werden soll, aufgewickelt wird und dieser Klebstoff in einer ersten Wicklungslage mit der Rückseite des Trägerbands einer weiter innen oder weiter außen liegenden Lage in Kontakt kommt. Beim Abwickeln steht der Klebstoff dann im Wesentlichen unbeeinträchtigt für eine Befestigung an der Windel bzw. als Verschluss zur Verfügung.

Statt einer Beschichtung können auch andere Maßnahmen vorgesehen sein, um ein Oberfläche des Trägerbandes oder des Release-Tapes klebstoffabweisend auszugestallten. So ist es beispielsweise denkbar, die physikalischen Eigenschaften des klebstoffabweisend auszugestaltenden Materials dieser Oberfläche gezielt zu verändern. Ebenso ist eine chemische Behandlung dieses Materials oder eine gezielte Auswahl dieses Materials in Abweichung von sonst üblichen Materialen denkbar, um die Oberfläche klebstoffabweisend auszubilden.

Das klebende Verhalten einer Klebstoffbeschichtung bzw. eines Klebstoffes in Bezug auf verschiedene Oberflächen bzw. Materialien, die hiermit in Kontakt gebracht werden bzw. werden können, ist hinlänglich bekannt und wird auch regelmäßig intensiv untersucht. Insofern stehen verschiedenste Möglichkeiten zur Verfügung, einen Klebstoff bzw. eine entsprechende Beschichtung derart zu modifizieren, dass die haftenden bzw. klebenden Eigenschaften an die jeweiligen Bedürfnisse angepasst sind. Ebenso stehen verschiedenste Möglichkeiten zur Verfügung die entsprechende Oberfläche klebstoffabweisend oder besonders klebstofffreundlich auszubilden, um auf diese Weise die haftenden bzw. klebenden Eigenschaften an die jeweiligen Bedürfnisse anzupassen. Durch geeignetes Zusammenspiel dieser Maßnahmen ist es insbesondere auch möglich, die entsprechenden Komponenten eines klebend wirksamen Verschlusses oder eines klebend wirksamen Zusatzverschlusses derart einzustellen, dass diese unlösbar aneinander haften bzw. ausreichend aber lösbar aneinander haften oder leicht lösbar aneinander haften.

Es sei festgehalten, dass der Begriff "unlösbar befestigt" einerseits eine lediglich durch Zerstörung zu trennende Befestigung oder andererseits eine unter normalen Betriebsbedingungen nicht zu öffnende Verbindung umfasst. So ist es denkbar, dass durch aufwändige Maßnahmen, wie thermische oder chemische Einwirkungen oder auch durch mechanische Eingriffe, wie Kratzen oder Schälen oder Knibbeln, eine als unlösbar bezeichnete Befestigung gelöst werden kann, solange derartige Maßnahmen zu aufwändig sind, um ein schnelles Lösen einer Verbindung unter Betriebsbedingungen, wenn eine Windel angelegt oder das Windelverschlussband neu justiert bzw. geöffnet wird, zu ermöglichen.

Je nach konkreter Umsetzung der Erfindung kann die unlösbare Befestigung eines Trägerbandes an der Windel auf der Windelausseite oder auf der Windelinnenseite erfolgen. Ebenso ist es denkbar, dass das Trägerband ggf. gemeinsam mit anderen Baugruppen des Windelverschlussbands, wie beispielsweise gemeinsam mit dem Release-Tape, einen Rand der Windel oder eines Windelohrs von zwei Seiten umgreift, wie dieses insbesondere bei sogenannten Y-Bonds hinlänglich bekannt ist. Ebenso ist es denkbar, dass das Trägerband ggf. gemeinsam mit weiteren Baugruppen des Windelverschlussbandes beidseits von Baugruppen der Windel, beispielsweise von einem Backsheet und einem Frontsheet, eingeschlossen an der Windel mit seiner Befestigungsseite unlösbar befestigt ist. Dementsprechend sind dann auch entsprechende Befestigungsmittel, wie Klebstoffe, Schweißungen, Prägungen oder ähnliches an dem Trägerband und den übrigen im Befestigungsbereich des Windelverschlussbands unlösbar mit der Windel verbundenen Baugruppen vorgesehen. Insofern können insbesondere eine entsprechende Klebstoffschicht oder sonstige Befestigungsmittel auf einer, der anderen oder beiden Seiten des Trägerbandes oder sonstiger Bänder im Befestigungsbereich des Windelverschlussbandes vorgesehen sein.

Ebenso können die Baugruppen der Verschlüsse, die an der Benutzerseite des Trägerbandes vorgesehen sind, Vorder- oder Rückseitig vorgesehen sein, je nach letztlicher Befestigungsausrichtung sowie etwaiger Umfaltungen des Trägerbandes bzw. des Windelverschlussbandes an der Windel, wobei hierbei im Sinne einer ordnungsgemäßen Funktionsweise darauf geachtet wird, dass der zweite Teil des mechanischen Verschlusses bestimmungsgemäß ausreichend unkompliziert an der Windel befestigt werden kann.

Es sei betont, dass ggf. das Windelverschlussband und die Windel bzw. das Windelohr einer Windel auch einstückig ausgebildet sein können. Hierbei kann insbesondere das Windelohr dementsprechend einen weiblichen Teil des mechanischen Verschlusses tragen oder sogar bilden, wobei dann der männliche Teil dort dementsprechend auf dieser Oberfläche einhakend befestigt mittels einer Klebeseite nach außen angeordnet ist.

Vorliegende Erfindung eignet sich für Windeln jeglicher Art, insbesondere für Erwachsenen- bzw. Inkontinenzwindeln aber auch Babywindeln. In diesem Zusammenhang sei betont, dass vorliegend der Begriff "Windeln" jegliche Art von absorbierenden Hygieneartikeln, wie beispielsweise auch Damenbinden bzw. Slipeinlagen oder ähnliches, umfasst, solange eine Verbindung zwischen zwei Baugruppen, seien die Windelohren einer Windel mit dem Windelkörper oder sei es eine Slipeinlage in einem Slip, wobei die Klebstoffbeschichtung des klebend wirksamen Verschlusses zunächst an dem Slip befestigt und zum Wiederöffnen der zweite Teil des zugehörigen mechanischen Verschlusses an dem Slip verbleibt, wieder öffenbar vorgesehen sein soll.

Es versteht sich, dass in anderen Ausführungsformen die Klebstoffbeschichtung auch offen verbleiben oder aber mit einem einfachen Deckblatt, welches vor Benutzung abgezogen wird, kaschiert sein kann.

Die Merkmale der vorstehend bzw. in den Ansprüchen beschriebenen Lösungen können gegebenenfalls auch kombiniert werden, um die Vorteile entsprechend kumuliert umsetzen zu können.

Weitere Vorteile, Ziele und Eigenschaften vorliegender Erfindung werden anhand nachfolgender Beschreibung von Ausführungsbeispielen erläutert, die insbesondere auch in anliegender Zeichnung dargestellt sind. In der Zeichnung zeigen:

| | |
|---|---|
| Figur 1 | einen schematischen Querschnitt durch ein Windelverschlussband vor seiner Befestigung an einer Windel; |
| Figur 2 | das Windelverschlussband nach Figur 1 nach seiner Befestigung an einer Windel einem erstmaligen Schließen des Windelverschlusses; |
| Figur 3 | das Windelverschlussband nach Figuren 1 und 2 bei Verschließen der Windel; |
| Figur 4 | einen schematischen Querschnitt durch ein weiteres Windelverschlussband in ähnlicher Darstellung wie Figur 3; |
| Figur 5 | einen schematischen Querschnitt durch ein weiteres Windelverschlussband in ähnlicher Darstellung wie Figur 1; |
| Figur 6 | das Windelverschlussband nach Figur 5 in ähnlicher Darstellung wie Figuren 3 und 4; |
| Figur 7 | einen schematischen Querschnitt durch ein weiteres Windelverschlussband in ähnlicher Darstellung wie Figuren 1 und 5; |
| Figur 8 | das Windelverschlussband nach Figur 7 in ähnlicher Darstellung wie Figuren 3, 4 und 6; |
| Figur 9 | eine Aufsicht auf das Target der Windelverschlussbänder nach Figuren 1 bis 8; |
| Figur 10 | eine Aufsicht auf ein weiteres Target in ähnlicher Darstellung wie Figur 9; |
| Figur 11 | eine Aufsicht auf ein weiteres Target in ähnlicher Darstellung wie Figuren 9 und 10; |
| Figur 12 | eine Seitenansicht des Targets nach Figur 10; |
| Figur 13 | eine Seitenansicht eines weiteren Targets in ähnlicher Darstellung wie Figur 12; |
| Figur 14 | eine Seitenansicht eines weiteren Targets in ähnlicher Darstellung wie Figuren 12 und 13; |
| Figur 15 | einen schematischen Querschnitt durch ein Windelverschlussband mit dem Target nach Figur 13 vor seiner Befestigung an einer Windel in ähnlicher Darstellung wie Figuren 1, 5 und 7; |
| Figur 16 | eine scheibenförmige Rolle von Bandmaterial zur Herstellung von Windelverschlussbändern in schematischer Darstellung; und |
| Figur 17 | eine kreuzgewickelte Spule von Bandmaterial zur Herstellung von Windelverschlussbändern in schematischer Darstellung. |

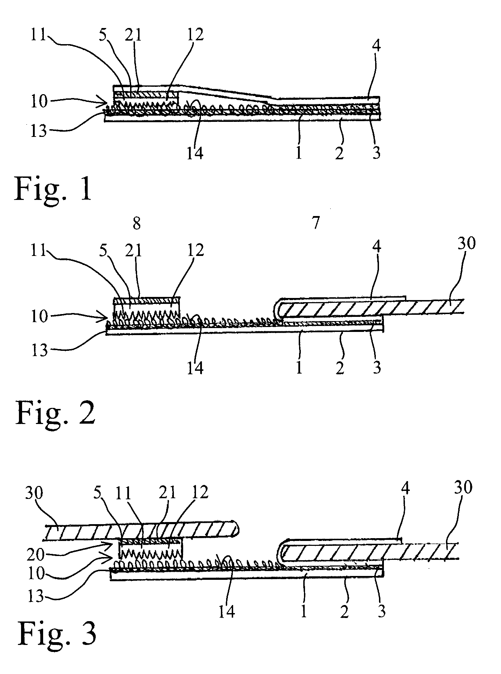

Das in Figuren 1 bis 3 dargestellte Windelverschlussband umfasst ein Trägerband 1 bestehend aus einer Folienlage 2 und einer Gewirkelage 3, auf welche an einer Seite, welche später die Befestigungsseite 7 (siehe Figur 2) bilden soll, ein Release-Tape 4 aufkaschiert ist. An der anderen Seite, welche später die Benutzerseite 8 (siehe Figur 2) bildet, sind die beiden Teile eines mechanischen Verschlusses 10, ein männlicher Teil 11 und ein weiblicher Teil 13, angeordnet, wobei bei diesem Ausführungsbeispiel der männliche Teil 11 aus einem Flicken aus Hakenmaterial 12 und der weibliche Teil 13 aus dem Schlaufenmaterial 14 der Gewirkelage 3 gebildet ist. In alternativen Ausführungsformen kann statt eines Gewirkes zum Bereitstellen der Gewirkelage 3 ein Gewebe oder ein Non-Woven zur Anwendung kommen, um eine entsprechende Lage bereitzustellen, wobei es sich versteht, dass eine derartige, ein Gewirke, eine Gewebe oder eine sonstiges textiles Flächenmaterial, wie ein Non-Woven, umfassende Lage auch als Laminat bzw. ihrerseits aus mehreren Lagen bestehend ausgebildet sein kann.

Auf der Rückseite des männlichen Teils 11, also auf der den Haken des Hakenmaterials 12 sowie dem weiblichen Teil 13 abgewandten Seite, ist eine für einen klebend wirksamen Verschluss 20 (siehe Figur 3) geeignete Klebstoffbeschichtung 21 vorgesehen, welche ihrerseits ebenfalls mit dem Release-Tape 4 kaschiert ist. Bei diesem Ausführungsbeispiel weist das Release-Tape 4 auf seiner dem Trägerband 1 abgewandten Seite ebenfalls eine nicht näher dargestellte Klebstoffbeschichtung auf, die als solchen jedoch bereits aus dem Stand der Technik bekannt ist.

Darüber hinaus ist die dem Trägerband 1 zugewandte Seite des Release-Tapes 4 im Bereich der Klebstoffbeschichtung 21 mit einer klebstoffabweisenden Beschichtung beschichtet, so dass, wie nachfolgend noch im Detail erläutert werden wird, die Klebstoffbeschichtung 21 ohne substantielle Beschädigung und mit vertretbar geringem Aufwand von dem Release-Tape 4 entfernt werden kann.

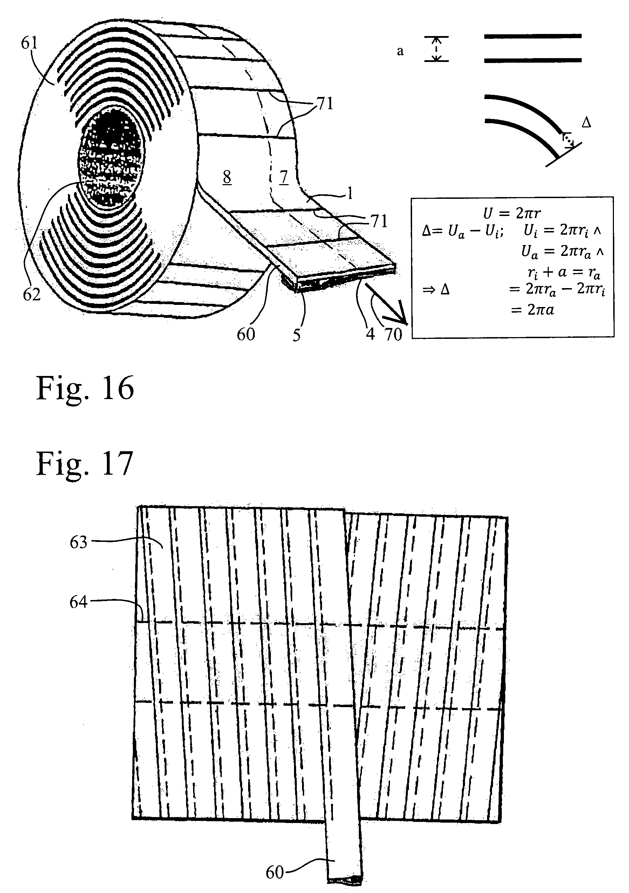

In der in Figur 1 schematisch dargestellten Ausrichtung und Anordnung kann das Windelverschlussband als Bahnenware ohne Weiteres ein- oder mehrnutzig aufgerollt werden, wie beispielhaft in Figuren 16 und 17 dargestellt, wobei die Bahn in der Darstellung nach Figur 1 dann vorzugsweise senkrecht zur Papierebene erstreckt ist. Dieses wäre dann auch dementsprechend die Maschinenrichtung.

Um ein Abwickeln zu erleichtern kann ggf. die dem Release-Tape 4 abgewandte Seite des Trägerbands 1 klebstoffabweisend beschichtet sein. Vor bzw. bei dem Applizieren des Windelverschlussbandes an der Windel werden ggf. zunächst die verschiedenen Nutzen entlang der Maschinenrichtung getrennt und quer zur Maschinenrichtung einzelne Streifen als Windelverschlussbänder abgetrennt. Hierdurch folgen Windelverschlussbänder mit einer Länge bzw. mit einer Längserstreckungsrichtung, welche von der Benutzerseite 8 zur Befestigungsseite 7 reicht und mithin senkrecht zur Maschinenrichtung ausgerichtet ist, und mit einer hierzu senkrecht liegenden Querrichtung, welche parallel zur Maschinenrichtung und dementsprechend senkrecht zur Papierebene der Zeichnung ausgerichtet ist.

Nach dem Abtrennen können die einzelnen Windelverschlussbänder an einem Windelkörper 30, beispielsweise an einem dort zu findenden Windelohr oder einer sonstigen Kante appliziert werden, indem das gesamte Band um diese Kante herum gefaltet wird, so dass das Release-Tape 4 den Windelkörper 30 an seiner Kante entsprechend umgibt.

In dieser Stellung wird die Windel mit dem Windelverschlussband vorzugsweise ausgeliefert, so dass die Klebstoffbeschichtung 21 weiterhin auf dem Release-Tape 4 ruht und das Windelverschlussband in einer Bereitstellungsposition bereitsteht, in welcher es ohne Weiteres zum Verschließen der Windel an seinem Benutzerende 8 ergriffen und in die Konfiguration nach Figur 2 gestreckt werden kann.

Die klebstoffabweisende Beschichtung des Release-Tapes 4 ist hierbei derart gewählt, dass ein Entfernen der Klebstoffbeschichtung von dem Release-Tape 4 ohne Weiteres möglich ist und dennoch die Klebstoffbeschichtung 21 in der Bereitstellungsposition ausreichend stabil auf dem Release-Tape 4 verbleibt.

In der Konfiguration nach Figur 2 kann das Windelverschlussband ohne Weiteres als Windelverschluss genutzt werden, indem die Klebstoffbeschichtung 21 zur Ausbildung eines klebend wirksamen Verschlusses 20 ebenfalls auf dem Windelkörper appliziert wird, wie dieses in Figur 3 dargestellt ist. Der Klebstoff der Klebstoffbeschichtung 21 ist hierbei derart gewählt, dass er ausreichend fest auf dem Windelkörper 30 haftet, wie dieses hinreichend bereits aus dem Stand der Technik bekannt ist. Für ein Öffnen und Wiederverschließen der Windel kann nunmehr ohne Weiteres der mechanische Verschluss 10 getrennt werden, so dass der männliche Teil 11, der mithin ein Target 5 bildet, aufgrund der Klebstoffbeschichtung 21 an dem Windelkörper 30 verbleibt. Zum Wiederverschließen wird der weibliche Teil 13 bzw. das Schlaufenmaterial 14 oder die Gewirkelage 3 wieder in Kontakt mit dem männlichen Teil 11 bzw. dem Target 5 gebracht und der mechanische Verschluss 10 geschlossen. Hierbei versteht es sich, dass eine Repositionierung des weiblichen Teils 13 in Bezug auf den männlichen Teil 11 ohne Weiteres im Rahmen der offenen Oberfläche der Gewirkelage 3 bzw. des offen zugänglichen Schlaufenmaterials möglich ist. Insbesondere kann die Windel ohne Weiteres ein wenig strammer angelegt werden.

Wie unmittelbar ersichtlich, ist der weibliche Teil 13 des mechanischen Verschlusses 10 über die gesamte Länge des Trägerbands 1 vorgesehen, wobei im Bereich der permanenten Befestigung des Release-Tapes 4 an der Gewirkelage 3 der weibliche Teil 13 letztlich nicht mehr funktionabel ist. In diesem Zusammenhang sei betont, dass an dieser Stelle bzw. in diesem Bereich ohne Weiteres auch auf eine Gewirkelage 3 verzichtet werden kann, solange im freien Bereich des Trägerbands 1 noch entsprechend Schlaufenmaterial 14 bzw. weiblicher Teil 13 zu finden ist.

Die Verbindung zwischen dem Trägerband 1 und dem Release-Tape 4 kann letztlich auf verschiedene Weise folgen. Beispielsweise kann hier eine Verschweißung vorgesehen sein. Ebenso kann auch eine Klebstoffverbindung vorgesehen sein, wobei, wenn auch dieser Bereich des Release-Tapes 4 mit einer klebstoffabweisenden Schicht beschichtet ist, ein Klebstoff gewählt wird, der hierdurch nur unwesentlich beeinträchtigt wird, so dass nach wie vor eine ausreichend stabile Verbindung zwischen Trägerband 1 und Release-Tape 4 gewährleistet ist.

Des Weiteren versteht es sich, dass ggf. auch das Release-Tape 4 auf seiner der Windel bzw. dem Windelkörper 30 abgewandten Seite eine Gewirkelage oder eine Lage mit einer anders ausgestalteten textilen Oberfläche, wie beispielsweise ein Gewebe oder ein Non-Woven aufweisen kann oder sogar zur Gänze aus einem Gewirke, oder alternativ aus einem anderen entsprechenden textilen Material, wie aus einem Gewebe oder einem Non-Woven, gebildet sein kann, so dass eine Repositionierung des mechanischen Verschlusses 10 auch im Bereich des Release-Tapes 4 möglich sein kann. Bei einer derartigen Ausgestaltung ist ggf. das Gewirke bzw. die entsprechende textile Oberfläche im Bereich des Release-Tapes 4 in geeigneter Weise klebstoffabweisend zu beschichten, was beispielsweise durch das Auftragen oder anderweitige Aufbringen von Silikon oder anderen klebstoffabweisenden Stoffen geschehen kann. Ebenso versteht es sich, dass ggf. auch eine dem Hakenmaterial 12 zugewandte Seite des Windelkörpers 30 bzw. eine dem männlichen Teil 11 zugewandte Seite des Windelkörper 30 für eine Repositionierung genutzt werden kann, wenn die Oberfläche des Windelkörpers 30 für einen Verschluss mit dem männlichen Teil 11 geeignet ist.

Es versteht sich des Weiteren, dass in einer abgewandelten Ausführungsform das Trägerband 1 auch einlagig ausgebildet sein kann, so dass auf eine separate Folienlage verzichtet wird. Ebenso können selbstverständlich noch weitere diverse Lagen im Trägerband 1 vorgesehen sein.

Darüber hinaus ist es auch denkbar, auf ein umgefaltetes Release-Tape 4 zu verzichten. Beispielsweise kann ein entsprechendes Release-Tape 4 lediglich im Bereich der Klebstoffbeschichtung 21 (siehe Figur 1) vorgesehen sein, während das Trägerband 1 auf andere Weise befestigungsseitig an der Windel befestigt wird. Auch ein derartiges kleines Release-Tape kann beim Umfalten des Trägerbandes 1 entsprechend auf der Innenseite eines Windelkörpers 30 befestigt werden.

Ebenso kann auf eine derartig umgefaltete Bereitstellungsposition verzichtet werden, indem das Release-Tape 4 auf seiner dem Hakenmaterial 12 abgewandten Seite keine Klebstoffbeschichtung aufweist, so dass das Windelverschlussband an seiner Benutzerseite 8 verhältnismäßig frei umherfliegt oder anderweitig in einer Bereitstellungsposition gehalten wird. Gegebenenfalls kann auf ein entsprechendes Release-Tape oder ein sonstiges, die Klebstoffbeschichtung 21 kaschierendes Tape verzichtet werden, wenn durch andere geeignete Maßnahmen die Klebstoffbeschichtung geschützt wird oder aber die Klebstoffbeschichtung 21, solange die Windel in der Verpackung ist, gegen Verschmutzung und versehentlichen Verkleben mit dem Windelkörper unempfindlich ausgestaltet ist.

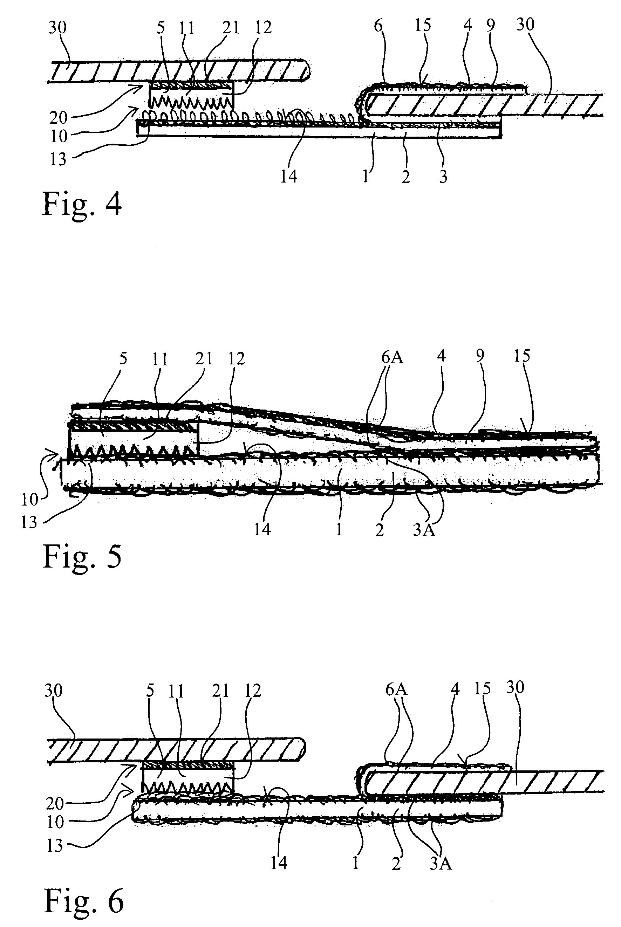

Das in Figur 4 dargestellte Windelverschlussband entspricht im Wesentlichen dem Windelverschlussband nach Figuren 1 bis 3, wobei jedoch das Release-Tape 4 ebenfalls eine Gewirkelage 6 aufweist, sodass das Target 5 beim Repositionieren auch dort angebracht werden und mithin die Variabilität für eine Wiederverschließen dementsprechend erhöht werden kann, in dem der mechanische Verschluss 10 hinsichtlich des weiblichen Teils 13 nicht nur das Schlaufenmaterial 14 sondern weiteres Schlaufenmaterial 15 der Gewirkelage 6 zur Verfügung hat.

Damit die Klebstoffbeschichtung 21 an dem die Gewirkelage 6 umfassenden Release-Tape 4 nicht unnötig korrumpiert wird, weist das Release-Tape 4 an der entsprechenden Stelle eine Silikonbeschichtung auf, wobei an dieser Stelle - je nach konkreter Umsetzung - auch andere klebstoffabweisende Vorkehrungen getroffen sein können. Je nach Wahl der Gewirkelage 6 kann auf eine derartige klebstoffabweichende Beschichtung auch ganz verzichtet werden.

Das in Figuren 5 und 6 dargestellte Ausführungsbeispiel entspricht im Wesentlichen dem Ausführungsbeispiel nach Figur 4, wobei jedoch das Trägerband 1 und das Release-Tape 4 statt jeweils einer Folienlage 2, 9 und einer Gewirkelage 3, 6 eine mit Non-Woven 3A, 6A beschichtete Folie umfassen, sodass auch dort das Release-Tape 4 einen weiblichen Teil 13 des mechanischen Verschlusses 10 durch Schlaufenmaterial 15 bereit stellt. Hierbei versteht es sich, dass ggf. auch auf das Non-Woven 6A des Release-Tapes 4 verzichtet werden kann, um einen Windelverschluss, welcher dem Ausführungsbeispiel nach Figuren 1 bis 3 entspricht, bereitzustellen. Ebenso ist es denkbar, das Release-Tape 4 lediglich auf der der Klebstoffbeschichtung 21 zugewandten Seite mit dem Non-Woven 6A zu versehen, um auf diese Weise einen äußerst sicheren Sitz einer Klebstoffbeschichtung zu dem Windelkörper 30 hinsichtlich des Release-Tapes 4 zu gewährleisten.

Bei diesem Ausführungsbeispiel sind die Folienlagen 2, 9 thermoelastisch ausgebildet, wobei in an sich bekannter Weise bestimmte Bereiche beispielsweise durch Vorstrecken oder sonstige an sich aus dem Stand der Technik bekannte Maßnahmen elastischer ausgebildet sind. Bei diesem Ausführungsbeispiel ist dieses der Bereich zwischen der Befestigungsseite 7 und der Benutzerseite 8. Auf diese Weise kann das Trägerband 1 ohne weiteres straffend verstreckt werden. Es versteht sich, dass in alternativen Ausführungsformen auch elastische Folienlagen 2, 9 oder insbesondere auch unterschiedliche Folienlagen 2, 9 zur Anwendung kommen können. Ebenso ist es denkbar, auf die Folienlage 2, 9 zu verzichten, solange die textilen Lagen, wie die Gewirkelage 3, 6 bzw. das Non-Woven 3A, 6A ausreichend eigenstabil sind.

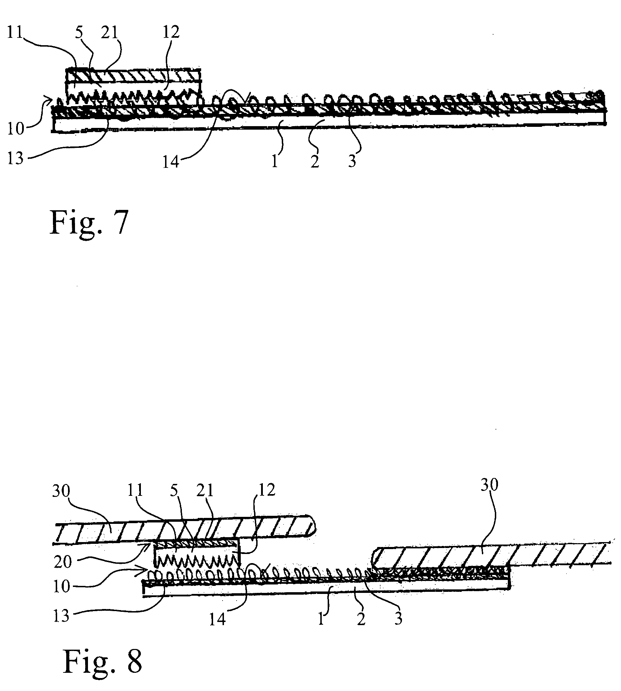

Bei dem in Figur 7 und 8 dargestellten Windelverschlussband wird auf ein Release-Tape 4 verzichtet. Im Übrigen entspricht dieses Windelverschlussband dem Ausführungsbeispiel nach Figuren 1 bis 3, sodass dementsprechend gleichwirkende Baugruppen auch gleich beziffert sind und auf doppelte Erläuterungen an dieser Stelle verzichtet wird. Dadurch, dass bei diesem Ausführungsbeispiel ebenso wie bei dem Ausführungsbeispiel nach Figuren 1 bis 3 die Rückseite der Folienlage 2 nicht textil kaschiert ist, kann der Klebstoff der Klebstoffbeschichtung 21 verhältnismäßig frei gewählt werden, ohne dass die Gefahr eines Verblockens besteht, wenn das entsprechende Bandmaterial von einer Spule oder Rolle abgewickelt werden soll. Gegebenenfalls kann jedoch auf der Rückseite auch eine klebstoffabweisende Beschichtung aufgebracht sein. Selbiges gilt auch, falls die Rückseite des Trägerbands textil ausgebildet ist, für das Trägerband, was einerseits durch eine zusätzliche dementsprechende Kaschierung, wie beispielsweise bei dem in Figuren 5 und 6 dargestellten Ausführungsbeispiel oder andererseits bei einem Trägerband, welches lediglich aus einer textilen Lage gebildet wird, dementsprechend umgesetzt sein kann.

Je nach konkreter Umsetzung kann bei den in Figur 7 und Figur 8 dargestellten Ausführungsbeispiel eine andere Faltung oder aber eine geeignete Beschichtung an dem Windelkörper 30 vorgesehen sein, um ein Verblocken des Klebstoffs der Klebstoffbeschichtung 21 betriebssicher zu vermeiden, bevor dieser zur Bildung des Targets 5 auf der Windel abgesetzt wird. Der Vorteil dieser Ausführungsform liegt insbesondere in der minimalen Anzahl an Komponenten und in der Tatsache, dass das Windelverschlussband äußerst dünn baut, sodass es in großen Gebinden ausgeliefert werden kann.

Das in den Ausführungsbeispielen nach Figuren 1 bis 8 zur Anwendung kommende Target 5 weist als männlichen Teil 11 des mechanischen Verschlusses 10 jeweils einen Flicken aus Hakenmaterial 12 auf, welcher lediglich aus einem einzigen Trägerelement 40 für die männlichen Verschlusselemente 41, nämlich die Haken des Hakenmaterials 12, besteht. Hierbei ist das Trägerelement 40 einstückig ausgebildet und die einzelnen Haken ragen als männliche Verschlusselemente 41 ebenfalls einstückig an dem Trägerelement 40 ausgebildet hervor.

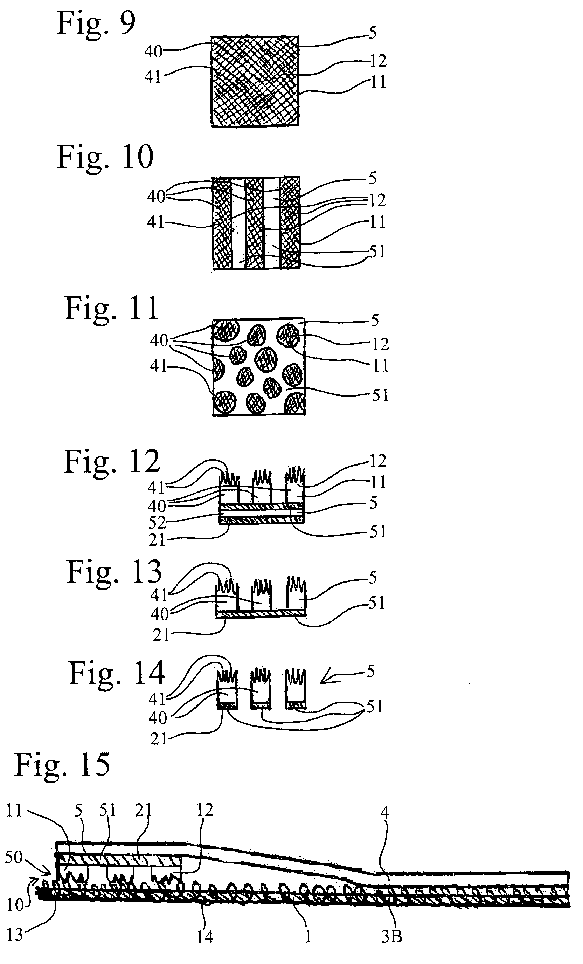

Eben solcher Art ausgebildete Trägerelemente 40 können auch mehrstückig zur Bildung des Targets 5 genutzt werden, wie in den Figuren 10 bis 15 exemplarisch dargestellt.

So weist das Target 5 des Ausführungsbeispiels nach Figur 10 drei streifenförmige Trägerelemente 40 auf, welche - wie in Figuren 12 dargestellt - mittels einer Klebstoffbeschichtung 51 auf einer Trägerfolie 52 angebracht sind, deren Rückseite wiederum mit der Klebstoffbeschichtung 21 zur Bildung des klebend wirksamen Verschlusses 20 beschichtet ist. Dieses bedingt einerseits eine wesentlich höhere Flexibilität des Targets 5, was schon an sich einen besseren Verschluss des mechanischen Verschlusses 10 gewährleistet. Auch werden hierdurch mehr männliche Verschlusselemente 41 bzw. Haken bereitgestellt, welche jeweils am Rand eines Trägerelements 40 vorhanden sind und augenscheinlich einen höheren Beitrag an Verschließungskräften bedingen als innenliegende männliche Verschlusselemente 41. Darüber hinaus verbleibt zwischen den Trägerelementen 40 jeweils ein freier Bereich der Klebstoffbeschichtung 51, welcher zusätzlich zu dem mechanischen Verschluss 10 einen Zusatzverschluss 50 gewährleistet, der den mechanischen Verschluss 10 unterstützt. Hierbei versteht es sich, dass die Klebstoffbeschichtung 51 derart eingestellt ist, dass sie mit dem Schlaufenmaterial 14, 15 des weiblichen Teils 13 in ausreichendem Maße klebend wechselwirken und so insbesondere peel-off-Kräfte aufnehmen kann.

Wie in Figur 13 exemplarisch dargestellt, kann ggf. auch auf die Trägerfolie 52 verzichtet werden, insbesondere wenn der Klebstoff 51 ausreichend eigenstabil ist. Gegebenenfalls ist jedoch letzteres nicht zwingend von Nöten, wenn die männlichen Verschlusselemente 51 ohnehin für eine ausreichende Verbindungsstärke sorgen können. Insbesondere dann kann auf einen Zusatzverschluss 50 verzichtet werden, wie dieses auch bei den Ausführungsbeispielen nach Figuren 1 bis 9 der Fall ist, wobei ggf. trotz allem mehrere Trägerelemente 40 vorgesehen seien, wie dieses exemplarisch Figur 14 zeigt. Dementsprechend ist es auch denkbar, die Klebstoffbeschichtung 51 derart zu konfektionieren, dass sie lediglich mit den Trägerelementen 40 klebend wechselwirkt und eine Wechselwirkung mit dem Schlaufenmaterial 14, 15 letztlich unterbleibt bzw. von nur sehr untergeordneter Bedeutung ist.

Darüber hinaus versteht es sich, dass die Trägerelemente 40 nicht zwingend streifenförmig vorgesehen sein müssen, wie exemplarisch am Ausführungsbeispiel nach Figur 11 verdeutlicht. Hier sind inselartige Trägerelemente 40 vorgesehen, wobei ggf. auch andere geometrische Formen dementsprechend zur Anwendung kommen können.

Es versteht sich, dass die in den Figuren 10 bis 14 dargestellten Targets 5 entsprechend der in Figuren 1 bis 9 dargestellten Ausführungsbeispiele als Windelverschlussband umgesetzt werden können, wie exemplarisch für das in Figur 13 dargestellte Ausführungsbeispiele in Figur 15 gezeigt, wobei hier als Trägerband 1 ein ausreichend eigenstabiles Gewirke 3B zur Anwendung kommt, sodass auf eine separate Folienlage 2 verzichtet werden kann. Wegen der zeichnerischen Überhöhung ist ein Kontakt der Klebstoffbeschichtung 51 mit dem weiblichen Teil 13 des mechanischen Verschlusses 10 bzw. mit dem Schlaufenmaterial 14 der Figur 15 nicht entnehmbar, wobei in der Praxis jedoch die Höhe der männlichen Verschlusselemente 41, der Abstand zwischen den Trägerelementen 40 und die Flauschigkeit bzw. die natürliche Nachgiebigkeit des weiblichen Teils 13 des mechanischen Verschlusses 10 derart ausgewählt werden können, dass ein ausreichender Kontakt zwischen dem weiblichen Teil 13 bzw. dem Schlaufenmaterial 14 und der Klebstoffbeschichtung sichergestellt ist.

Letztlich kann das den Windelverschlussbändern nach Figuren 1 bis 15 zugrunde liegende Material 60 ohne weitere als Rolle 61 aufgewickelt werden, wie beispielhaft in Figur 16 dargestellt, wobei hierzu, der Einfachheit halber, das Material um einen Rollenkern 62 gewickelt wird, hier letztlich auch andere Maßnahmen möglich sind. Hierbei wird das Bandmaterial 60 entlang seiner Längserstreckungsrichtung 70 aufgewickelt, welche der oben genannten Maschinenrichtung entspricht und kann dann quer hierzu durchtrennt werden, wie beispielhaft durch die Linien 71 angedeutet. Dementsprechend liegt dann die Längsrichtung der Windelverschlussbänder parallel zu den Linien 71, während die Querrichtung der Windelverschlussbänder parallel zur Längserstreckungsrichtung 70 bzw. parallel zur Maschinenrichtung liegt.

Aufgrund der verhältnismäßig großen Dicke, also der räumlichen Dimension senkrecht zur Längserstreckungsrichtung 70 und senkrecht zu den Linien 71, welche letztlich durch einen Abstand a repräsentiert werden kann, kommt es mit jeder Wicklung in der Rolle 61 zu einem Versatz $\Delta$, welcher letztlich ab einer bestimmten Zahl an Wicklungen zu Verwerfungen führt, die ein ordnungsgemäßes Arbeiten mit dem entsprechenden Material 60 erheblich erschweren. Dieser Nachteil lässt sich dadurch minimieren, dass das Bandmaterial 60 in Form einer Kreuzwicklung 63 um einen Spulenkern 64 gewickelt wird, wie exemplarisch in Figur 17 dargestellt. Statt eines Spulenkerns 64 können an dieser Stelle auch andere Maßnahmen, wie eine geeignete Umfaltung oder ähnliches, zur Anwendung kommen. Hierbei versteht es sich, dass im Bereich der Seiten dieser Kreuzwicklung 63 die übereinanderliegenden Bandteile der einzelnen Lagen nicht zwingend unterschiedliche Neigungswinkel aufweisen müssen. Hier können über bestimmte Winkelgrade auch parallele Lagen zu finden sein, wobei die Richtungsänderung vorzugsweise so schnell vorgenommen wird, dass keine unnötigen Materialanhäufungen an den Seiten der Kreuzwicklung 63 zu finden sind.

Je nach konkreter Ausgestaltung des Bandmaterials 60 werden die einzelnen Wicklungen einer Lage nebeneinander oder überlappend angeordnet.

Bezugszeichenliste:

| | | | |
|---|---|---|---|
| 1 | Trägerband | 20 | klebend wirksamer Verschluss |
| 2 | Folienlage | 21 | Klebstoffbeschichtung |
| 3 | Gewirkelage | 30 | Windelköper |
| 3A | Non-Woven | 40 | Trägerelement |

(fortgesetzt)

| 4 | Release-Tape | 41 | männliches Verschlusselement (exemplarisch beziffert) |
| 5 | Target | | |
| 6 | Gewirkelage | 50 | Zusatzverschluss |
| 6A | Non-Woven | 51 | Klebstoffbeschichtung |
| 7 | Befestigungsseite | 52 | Trägerfolie |
| 8 | Benutzerseite | 60 | Bandmaterial |
| 9 | Folienlage | 61 | Rolle |
| 10 | mechanischer Verschluss | 62 | Rollenkern |
| 11 | männlicher Teil | 63 | Kreuzwicklung |
| 12 | Hakenmaterial | 64 | Spulenkern |
| 13 | weiblicher Teil | 70 | Längserstreckungsrichtung |
| 14 | Schlaufenmaterial | 71 | Linien |
| 15 | Schlaufenmaterial | | |

## Patentansprüche

1. Windelverschlussband mit einer Befestigungsseite (7) und mit einer Benutzerseite (8) sowie mit einem von der Befestigungsseite (7) zur Benutzerseite (8) reichenden Trägerband (1), wobei an der Benutzerseite (8) des Trägerbandes (1) ein erster Teil eines aus einem männlichen und einem weiblichen Teil (11, 13) bestehenden mechanischen Verschlusses (10) unlösbar befestigt ist, wobei mit diesem ersten Teil der zweite Teil des mechanischen Verschlusses (10) verbunden ist und wobei an dem zweiten Teil an der von dem ersten Teil abweisenden Seite eine Klebstoffbeschichtung (21) eines klebend wirksamen Verschlusses (20) angeordnet ist, **dadurch gekennzeichnet, dass** der zweite Teil des mechanischen Verschlusses (10) der männliche Teil (11) ist und der erste Teil des mechanischen Verschlusses (10) der weibliche Teil (13) und der weibliche Teil (13) des mechanischen Verschlusses (10) größer als der männliche Teil (11) ausgebildet ist.

2. Windelverschlussband nach Anspruch 1, **dadurch gekennzeichnet, dass** das Trägerband (1) den weiblichen Teil (13) des mechanischen Verschlusses (10) bildet.

3. Windelverschlussband nach einem der vorstehenden Ansprüche, **dadurch gekennzeichnet, dass** der männliche Teil (11) des mechanischen Verschlusses (10) wenigstens zwei getrennte Trägerelemente (40) für männliche Verschlusselemente (41) umfasst.

4. Windelverschlussband nach einem der vorstehenden Ansprüche, **dadurch gekennzeichnet, dass** zusätzlich zu dem mechanischen Verschluss (10) ein klebend zwischen männlichem und weiblichem Teil (11, 13) wirksamer Zusatzverschluss (50) vorgesehen ist.

5. Windelverschlussband nach Anspruch 4, **dadurch gekennzeichnet, dass** neben dem männlichen Teil (11) des mechanischen Verschlusses (10) eine Klebstoffbeschichtung (51) des klebend wirksamen Zusatzverschlusses (50) vorgesehen ist.

6. Windelverschlussband nach einem der vorstehenden Ansprüche, **dadurch gekennzeichnet, dass** der weibliche Teil (13) des mechanischen Verschlusses (10) zumindest über die halbe Länge des Trägerbands (1) vorgesehen ist.

7. Windelverschlussband nach einem der vorstehenden Ansprüche, **dadurch gekennzeichnet, dass** das Trägerband (1) elastisch ausgebildet ist oder einen elastischen Bereich aufweist.

8. Windelverschlussband nach einem der vorstehenden Ansprüche, **dadurch gekennzeichnet, dass** der klebend wirksame Verschluss (20) mittels eines Release-Tapes (4) abgedeckt ist, das auf seiner dem klebend wirksamen Verschluss (20) zugewandten Seite klebstoffabweisend und auf seiner dem klebend wirksamen Verschluss (20) abgewandten Seite klebend ausgebildet ist.

9. Windelverschlussband nach einem der vorstehenden Ansprüche, **dadurch gekennzeichnet, dass** eine dem mechanischen Verschluss (10) abgewandte Seite des Trägerbands (1) klebstoffabweisend ausgebildet ist.

10. Windelverschlussband nach einem der vorstehenden Ansprüche, **dadurch gekennzeichnet, dass** der klebend wirksame Verschluss (20) unlösbar schließt.

11. Bandmaterial (60) mit einer Längserstreckungsrichtung, welches durch Trennen quer zur Längserstreckungsrichtung (70), und gegebenenfalls durch Trennen in Längserstreckungsrichtung (70), zu Einzelnutzen in Form von Windelverschlussbändern nach einem der vorstehenden Ansprüche, bevorzugt ohne Verschnitt, aufteilbar ist.

12. Verfahren zum Verschließen und Wiederverschließen einer Windel mit einem Windelkörper und mit einem Windelverschlussband nach Anspruch 1 oder 2, **dadurch gekennzeichnet, dass** der männliche Teil (11) des mechanischen Verschlusses (10) sowie die an ihm angeordnete Klebstoffbeschichtung (21) ein Target (5) bilden, welches nach dem erstmaligen Verschließen an dem Windelkörper (30) verbleibt und bei einem Wiederverschließen als Bestandteil des mechanischen Verschlusses (10) hierfür zur Verfügung steht, und dass, wenn der mechanische Verschluss (10) geöffnet wird, der männliche Teil (11) an dem Windelkörper (30) über die Klebstoffbeschichtung (21) verbleibt und anschließend der weibliche Teil (13) zum Wiederverschließen wieder an dem männlichen Teil (11) appliziert wird.

**Claims**

1. A nappy closing strip having a fastening side (7) and having a user side (8) and also having a carrier strip (1) reaching from the fastening side (7) to the user side (8), wherein a first part of a mechanical closure (10) consisting of a male and a female part (11, 13) is non-releasably fastened to the user side (8) of the carrier strip (1), wherein the second part of the mechanical closure (10) is connected to this first part, and wherein an adhesive coating (21) of an adhesively effective closure (20) is arranged on the second part on the side averted from the first part, **characterised in that** the second part of the mechanical closure (10) is the male part (11) and the first part of the mechanical closure (10) is the female part (13) and the female part (13) of the mechanical closure (10) is larger than the male part (11).

2. The nappy closing strip according to claim 1, **characterised in that** the carrier strip (1) forms the female part (13) of the mechanical closure (10).

3. The nappy closing strip according to any one of the preceding claims, **characterised in that** the male part (11) of the mechanical closure (10) comprises at least two separate carrier elements (40) for male closure elements (41).

4. The nappy closing strip according to any one of the preceding claims, **characterised in that**, in addition to the mechanical closure (10), an additional closure (50) which is adhesively effective between the male and female part (11, 13) is provided.

5. The nappy closing strip according to claim 4, **characterised in that**, besides the male part (11) of the mechanical closure (10), an adhesive coating (51) of the adhesively effective additional closure (50) is provided.

6. The nappy closing strip according to any one of the preceding claims, **characterised in that** the female part (13) of the mechanical closure (10) is provided at least over half the length of the carrier strip (1).

7. The nappy closing strip according to any one of the preceding claims, **characterised in that** the carrier strip (1) is elastic or has an elastic region.

8. The nappy closing strip according to any one of the preceding claims, **characterised in that** the adhesively effective closure (20) is covered by means of a release tape (4), which repels adhesive on its side facing towards the adhesively effective closure (20) and is adhesive on its side averted from the adhesively effective closure (20).

9. The nappy closing strip according to any one of the preceding claims, **characterised in that** a side of the carrier strip (1) averted from the mechanical closure (10) repels adhesive.

10. The nappy closing strip according to any one of the preceding claims, **characterised in that** the adhesively effective closure (20) closes non-releasably.

11. A strip material (60) having a direction of longitudinal extension, which strip material can be divided by separation transversely to the direction of longitudinal extension (70), and optionally by separation in the direction of longitudinal extension (70), into individual blanks in the form of nappy closing strips according to any one of the preceding claims, preferably without waste.

12. A method for closing and re-closing a nappy having a nappy body and having a nappy closing strip according to claim 1 or 2, **characterised in that** the male part (11) of the mechanical closure (10) and the adhesive coating (21) arranged thereon form a target (5) which remains on the nappy body (30) once said closure has been closed for the first time and, when the closure is re-closed, is available for this purpose as part of the mechanical closure (10), and **in that**, when the mechanical closure (10) is opened, the male part (11) remains on the nappy body (30) via the adhesive coating (21) and the female part (13) is then applied again to the male part (11) to re-close the closure.

**Revendications**

1. Ruban de fermeture de couche, avec une face de fixation (7) et avec une face utilisateur (8), ainsi qu'avec un ruban de support (1) allant de la face de fixation (7) vers la face utilisateur (8), sur la face utilisateur (8) du ruban de support (1) étant fixée une première partie d'une fermeture (10) mécanique constituée d'une partie mâle et d'une partie femelle (11, 13), la deuxième partie de la fermeture (10) mécanique étant reliée avec cette première partie, sur la deuxième partie, sur la face opposée à la première partie étant placé un revêtement d'agent adhésif (21) d'une fermeture (20) à effet adhésif, **caractérisé en ce que** la deuxième partie de la fermeture (10) mécanique est la partie mâle (11) et la première partie de la fermeture (10) mécanique est la partie femelle (13) et **en ce que** la partie femelle (13) de la fermeture (10) mécanique est conçue en étant plus grande que la partie mâle (11).

2. Ruban de fermeture de couche selon la revendication 1, **caractérisé en ce que** la bande de support (1) forme la partie femelle (13) de la fermeture (10) mécanique.

3. Ruban de fermeture de couche selon l'une quelconque des revendications précédentes, **caractérisé en ce que** la partie mâle (11) de la fermeture (10) mécanique comprend au moins deux éléments de support (40) séparés pour des éléments de fermeture (41) mâles.

4. Ruban de fermeture de couche selon l'une quelconque des revendications précédentes, **caractérisé en ce qu'**en supplément de la fermeture (10) mécanique, il est prévu une fermeture supplémentaire (50) agissant par adhésion entre les parties mâle et femelle (11, 13).

5. Ruban de fermeture de couche selon la revendication 4, **caractérisé en ce qu'**hormis la partie mâle (11) de la fermeture (10) mécanique, il est prévu un revêtement par agent adhésif (51) de la fermeture supplémentaire (50) agissant par adhésion.

6. Ruban de fermeture de couche selon l'une quelconque des revendications précédentes, **caractérisé en ce que** la partie femelle (13) de la fermeture (10) mécanique est prévue au moins sur la demi-longueur du ruban de support (1).

7. Ruban de fermeture de couche selon l'une quelconque des revendications précédentes, **caractérisé en ce que** le ruban de support (1) est conçu en étant élastique ou comporte une région élastique.

8. Ruban de fermeture de couche selon l'une quelconque des revendications précédentes, **caractérisé en ce que** la fermeture (20) agissant par adhésion est cachée par un ruban repositionnable (4) qui sur sa face dirigée vers la fermeture (20) agissant par adhésion est conçu en étant antiadhésif et sur sa face opposée à la fermeture (20) agissant par adhésion est conçu en étant adhésif.

9. Ruban de fermeture de couche selon l'une quelconque des revendications précédentes, **caractérisé en ce qu'**une face de la bande de support (1) opposée à la fermeture (10) mécanique est conçue en étant antiadhésive.

10. Ruban de fermeture de couche selon l'une quelconque des revendications précédentes, **caractérisé en ce que** la fermeture (20) agissant par adhésion ferme de manière inamovible.

11. Matière en bande (60) avec une direction d'extension longitudinale laquelle est divisible, de préférence sans chute, par sectionnement à la transversale de la direction d'extension longitudinale (70) et le cas échéant, par sectionnement dans la direction d'extension longitudinale (70) pour l'utilisation individuelle sous la forme de rubans de fermeture de couche selon l'une quelconque des revendications précédentes.

**12.** Procédé destiné à fermer et à refermer une couche, avec un corps de couche et avec un ruban de fermeture de couche selon la revendication 1 ou 2, **caractérisé en ce que** la partie mâle (11) de la fermeture (10) mécanique, ainsi que le revêtement d'agent adhésif (21) placé sur cette dernière forment une cible (5), qui après la première fermeture reste sur le corps (30) de la couche et lors d'une refermeture reste à disposition à cet effet, en tant qu'élément de la fermeture (10) mécanique et **en ce que** lorsque l'on ouvre la fermeture (10) mécanique, la partie mâle (11) reste sur le corps (30) de la couche par l'intermédiaire du revêtement d'agent adhésif (21) et **en ce qu'**ensuite, on réapplique la partie femelle (13) pour la refermeture de la partie mâle (11).

Fig. 1

Fig. 2

Fig. 3

Fig. 4

Fig. 5

Fig. 6

Fig. 7

Fig. 8

Fig. 9

40
41
5
12
11

Fig. 10

40
41
5
12
11
51

Fig. 11

40
41
5
12
11
51

Fig. 12

41
40
52
21
12
11
5
51

Fig. 13

41
40
21
5
51

Fig. 14

41
40
21
5
51

Fig. 15

11
50
10
13
5  51  21
12
14  1
4
3B

a

$\Delta$

$$U = 2\pi r$$
$$\Delta = U_a - U_i; \quad U_i = 2\pi r_i \wedge$$
$$U_a = 2\pi r_a \wedge$$
$$r_i + a = r_a$$
$$\Rightarrow \Delta \qquad = 2\pi r_a - 2\pi r_i$$
$$= 2\pi a$$

61

71

8    7

1

71

62

60    5    4 70

## Fig. 16

## Fig. 17

63

64

60

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19805576 A1
- EP 0832631 A2
- EP 1629813 A1
- US 2003130644 A1
- EP 0894448 A1
- WO 9827922 A1
- DE 69709244 T2
- EP 0813851 B1
- DE 69711244 T2
- EP 0941728 A2
- EP 0964665 B1
- EP 1000598 A1
- US 5019073 A
- US 5176670 A
- WO 2005074852 A1